(19) Europäisches Patentamt
European Patent Office
Office européen des brevets

(11) Publication number: **0 388 950 A2**

(12) **EUROPEAN PATENT APPLICATION**

(21) Application number: **90105433.8**

(51) Int. Cl.5: **C07D 405/06, A61K 31/44**

(22) Date of filing: **22.03.90**

(30) Priority: **22.03.89 ES 8901387**

(43) Date of publication of application:
**26.09.90 Bulletin 90/39**

(84) Designated Contracting States:
**AT BE CH DE DK FR GB GR IT LI LU NL SE**

(71) Applicant: **J. URIACH & CIA. S.A.**
**Degà Bahi, 59-67**
**E-08026 Barcelona(ES)**

(72) Inventor: **Bartroli, Javier**
**Vives i Tutó 55**
**ES-08034 Barcelona(ES)**
Inventor: **Anguita, Manuel**
**Rambla Volart 85**
**ES-08026 Barcelona(ES)**
Inventor: **Carceller, Elena**
**Aragón 117**
**ES-08015 Barcelona(ES)**

(74) Representative: **Zumstein, Fritz, Dr. et al**
**Zumstein & Klingseisen Patentanwälte**
**Bräuhausstrasse 4**
**D-8000 München 2(DE)**

(54) **New 1,3-dioxolan-4-yl derivatives of 2-picolylamine.**

(57) The present invention relates to new 1,3-dioxolan-4-yl derivatives of 2-picolylamine having the formula (I):

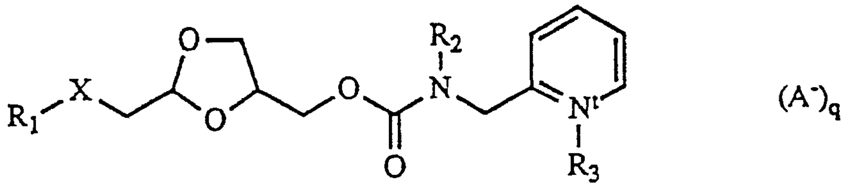

(I)

wherein:
$R_1$ represents an alkyl or phenylalkyl radical; $R_2$ represents hydrogen, $C_1$-$C_4$-acyl or $C_1$-$C_4$-alkoxycarbonyl; $R_3$ is either an electron pair, in which case t means nothing and q is zero, or $R_3$ is hydrogen or $C_1$-$C_4$-alkyl, in which case t is (+) and q=1; X is a single bond, an oxygen atom or a carbamoyloxy group; $A^-$ is a pharmaceutically acceptable anion. These compounds are PAF antagonists and, consequently, useful in the treatment of the diseases in which this substance is involved.

EP 0 388 950 A2

**New 1,3-dioxolan-4-yl derivatives of 2-picolylamine.**

The present invention relates to new 1,3-dioxolan-4-yl derivatives of 2-picolylamine with a potent antagonist activity of the platelet activating factor (PAF), together with a process for their preparation. The invention also relates to the pharmaceutical preparations which contain these compounds an their use in the treatment of the diseases in which PAF is involved, such as allergic and bronchial asthma, platelet aggregation disorders, septic shock, hypertension, etc.

The platelet activating factor (PAF) or (1-*O*-alkyl-2-acetyl-*sn*-glyceryl-3-phosphorylcholine), also called acetyl glyceryl ether phosphorylcholine (AGEPC) or PAF-acether, is a natural phospholipid synthesized by different cells (basophiles, macrophages, neutrophiles, platelets) and tissues (heart, lung and kidney) of the organism (Roubin et al. in "Lymphokines" Ed. E. Pick, Acad. Press. New York, p. 249, **1983**; Vargaftig et al, *Ann. N.Y. Acad. Sci.*, **1981**, 370, 119; Pinckard et al., *Int. Arch. Allergy Appl. Immun.*, **1981**, 66, 127).

PAF was described for the first time as a potent platelet aggregating agent (Benveniste et al., *J. Exp. Med.*, **1972**, 136) and later it was proved to have other biological activities *in vivo*, such as peripheral vasodilatation, increase of the vascular permeability, induction of bronchoconstriction and hyperreactivity of the respiratory tract (Mazzoni, et al. *J.Physiol.* **1985**, 365, 107P). PAF also produces immediate hypotension followed by pulmonary and renal hypertension in rats (Blank et al., *Biochem. Biophis. Res. Commun.*, **1979**, 90, 1194), guinea pigs (Feuerstein, et al., *Circul. Shock*, **1984**, 13, 255), rabbits (Muirhead et al., *Hypertension*, **1981**, 3, 107) and dogs (Otsuka, et al., *Prostaglandins Leukotrienes Med.*, **1985**, 19, 25), and it has been rated as the most potent ulcerogenic agent described until now.

Consequently, PAF is a mediator that is implicated in a large set of pathological processes such as asthma, septic shock, transplant rejection, thrombosis, ulceration, inflammation and renal diseases.

Even though its mechanism of action is still not known with precision, some studies show that the biological activities of PAF involve the existence of a specific receptor. Recently, one of these receptors has been isolated from human platelets and it has been identified as a protein with a molecular weight of 160.000 daltons (Nishihira et al., *Tohoku J. Exp. Med.*, **1985**, 147, 145). On the other hand, the capacity to inhibit the binding of $^{3}$H- PAF to their receptors is well correlated with the amount of PAF needed to provoke the *in vitro* and *in vivo* observed effects. These facts indicate that the compounds that act as specific antagonists of PAF could result of interest for the treatment of all those pathological processes related directly or indirectly with PAF.

According to the above mentioned activities, PAF antagonists have been investigated with the aim of developing new types of anti-shock, anti-inflammatory and anti-asthma agents. In particular, analogues of natural PAF's have been investigated in an attempt to find such PAF antagonists. These compounds are disclosed in patents EP 138559, JP 57/165394, JP 58/35116, JP 61 93191, EP 178261, and EP 210804, among others. Some non-phosphate glycerol derivatives have been shown to posess PAF antagonist activity, like, for example, those disclosed in patents EP 147768, and EP 254540. Patents EP 157609, EP 238202, EP 251827, EP 312041 and EP 312040 disclose compounds having the substituent depicted in figure 1, which also appears in the compounds of the present invention.

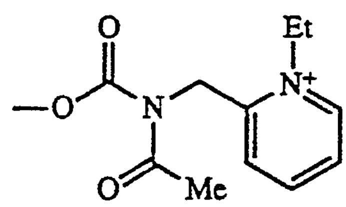

Figure 1

The compounds disclosed in the above-mentioned patents, however, are unsactisfactory for one or more of the following reasons: they lack sufficient PAF antagonist activity; they have short half-lives; their biological utilization is inadequate; their syntheses is too tedious.

Our invention ES 87 02901 describes 1,3-dioxolane derivatives , of formula,

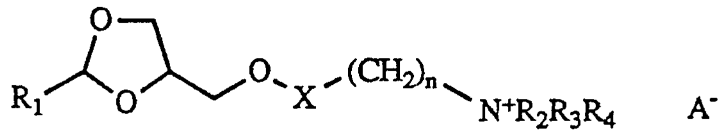

wherein $R_1$ represents a linear alkyl chain and X is a single bond, or a C=O, OC=O, NHCO, $NR_5CO$ or $OPO_2$ group, where $R_5$ is a lower alkyl or acyl group, n is 2-10 and $R_2R_3R_4N^+$ represents an heterocycle linked to the methylene chain by a quaternary nitrogen. In the present invention, the nitrogen is not directely linked to the methylene chain and belongs always to a pyridine ring as represented in figure 1.

Thus, the new compounds of the present invention not only are structurally different to the compounds described in the forementioned patents, but moreover, and quite surprisingly, they show a better antagonist activity of PAF and/or hypotensive action.

The compounds to which the present invention relates may be represented by the formula (**I**):

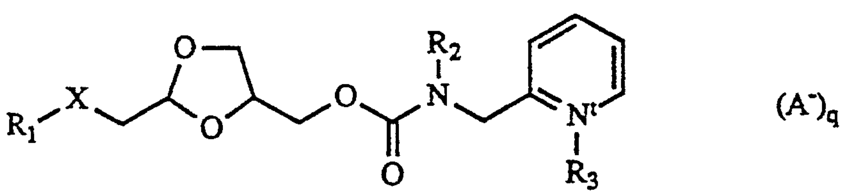

(I)

in which:

$R_1$ represents a $C_{10}$-$C_{24}$ alkyl, alkenyl or alkynyl group, a *tert*-butyl group, an aryl-$C_1$-$C_{12}$ alkyl or a cyclohexyl-$C_1$-$C_{12}$ alkyl group;

$R_2$ is hydrogen or a -C(=O)$R_4$ group, wherein $R_4$ represents a $C_1$-$C_6$ alkyl or $C_1$-$C_6$ alkoxy group;

X is a single bond or a -O-, -C(=O)O- or -$NR_5$C(=O)O- group, wherein $R_5$ is hydrogen, $C_1$-$C_4$-alkyl or $C_1$-$C_4$ acyl;

q is 0 or 1;

t is (+) when q=1, and t has no meaning when q=0;

$R_3$ is hydrogen or $C_1$-$C_6$ alkyl when q=1 and an electron pair when q=0;

$A^-$ is a pharmaceutically acceptable anion.

The invention also provides the use for the manufacture of a medicament for the treatment or prophylaxis of PAF-mediated illnesses in an animal, especially a mammal, which may be a human being, of at least one compound of formula (**I**)

The invention still further provides a pharmaceutical composition comprising an effective amount of at least one compound of formula (**I**) in admixture with a pharmaceutically acceptable carrier or diluent.

The invention also provides processes for preparing the compounds of the present invention, which in general terms comprise:

(a) reacting 1-benzyloxy-2,3-propanediol (**II**), with a compound of formula (**III**)

$R_1$-X-$CH_2$CHO (**III**)

[in which $R_1$ and X are as defined above] to give a compound of formula (**IV**)

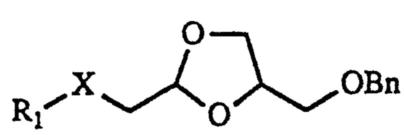

(IV)

[in which $R_1$ and X are as defined above] and reacting said compound of formula (**IV**) with hydrogen gas in the presence of a metal catalyst to give compound (**V**)

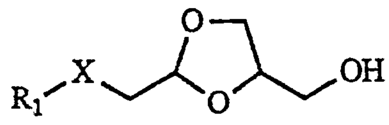

(V)

[in which $R_1$ and X are as defined above] and reacting said compound of formula (V) with a compound of formula (VI)

$Y'C(=O)Y''$ (VI)

[in which Y' and Y'' are good leaving groups] to give a compound of formula (VII)

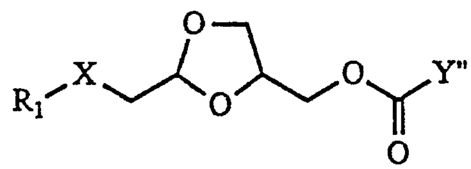

(VII)

[in which $R_1$, Y'' and X are as defined above] and reacting said compound of formula (VII) with 2-aminomethylpyridine to afford a compound of formula (Ia)

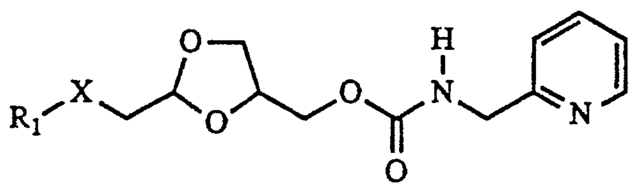

(Ia)

[in which $R_1$ and X are as defined above] and optionally reacting said compound of formula (Ia)with a compound of formula (VIII)

$ClC(=O)R_4$ (VIII)

[in which $R_4$ is as defined above] to give a compound of formula (Ib)

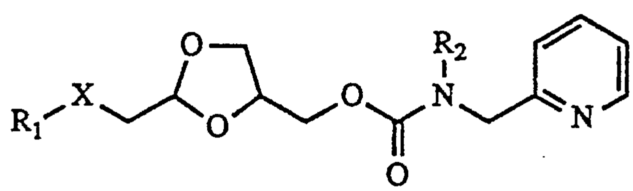

(Ib)

[in which $R_1$, $R_2$, and X are as defined above, but $R_2$ is different from hydrogen] and optionally reacting said compound (Ib) with a compound of formula (IX)

$R_3A$ (IX)

[in which $R_3$ and A is as defined above] to afford a compound of formula (Id)

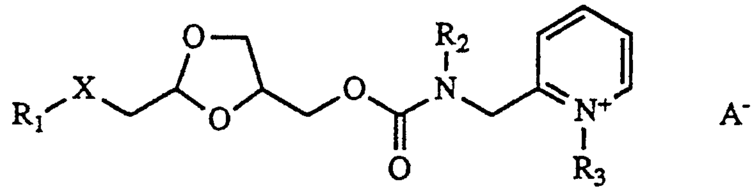

(Id)

[in which $R_1$, $R_2$, $R_3$, X, and A are as defined above, but $R_2$ is different from hydrogen];
or
(b) optionally reacting a compound of formula (**Ia**) with a compound of formula (**IX**) to give a compound of formula (**Ic**)

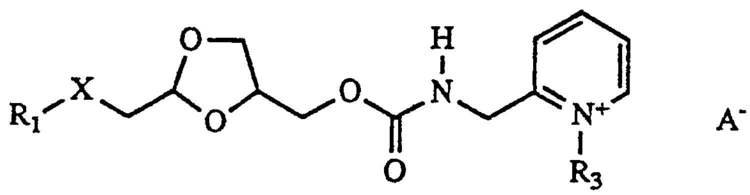

(Ic)

[in which $R_1$, $R_3$, X, and A are as defined above].
and
(c) optionally changing anion A by another pharmaceutically acceptable anion by means of ion-exchange chromatography or selective salt precipitation.

In the compounds of the present invention, where $R_1$ represents an alkyl group, this may be a straight or branched chain alkyl group containing from 10 to 24 carbon atoms, and examples include the *n*-decyl, *n*-undecyl, *n*-dodecyl, *n*-tridecyl, *n*-tetradecyl, *n*-pentadecyl, *n*-hexadecyl, *n*-heptadecyl,*n*-octadecyl, *n*-nonadecyl, *n*-eicosenyl groups, or a *tert*-butyl group, or 4-phenylbutyl, 3-phenylpropyl, 2-phenylethyl, phenylmethyl, 5-cyclohexylpentyl, 4-cyclohexylbutyl. 3-cyclohexylpropyl, 2-cyclohexylethyl and cyclohexylmethyl groups, of which the *n*-tetradecyl, *n*-pentadecyl, *n*-hexadecyl, *n*-heptadecyl, *n*-octadecyl, *n*-nonadecyl, 3-phenylpropyl and cyclohexylmethyl groups are preferred, and the *n*-pentadecyl, *n*-hexadecyl, *n*-heptadecyl, *n*-octadecyl groups are most preferred.

Where $R_2$ represents hydrogen or a group of formula $-C(=O)R_4$, and $R_4$ represents a $C_1$-$C_6$ alkyl group or $C_1$-$C_6$ alkoxy group, $R_4$ may be a straight or branched chain alkyl or aikoxy group containing 1 to 6 carbon atoms, and examples include methyl, ethyl, *n*-propyl, *iso*-propyl, *n*-butyl, *iso*-butyl, *tert*-butyl, *n*-pentyl, *iso*-pentyl, *neo*-pentyl, *n*-hexyl, methoxy, ethoxy, *n*-propoxy, *iso*-propoxy, *iso*-butoxy, *tert*-butoxy, *n*-pentoxy, *iso*-pentoxy and *n*-hexoxy, of which the methyl and ethoxy groups are more preferred, and the methyl group is most preferred.

Where $R_3$ represents hydrogen or a $C_1$-$C_6$ alkyl group this may be a straight or branched chain alkyl group containing 1 to 6 carbon atoms, and examples include methyl, ethyl, *n*-propyl, *iso*-propyl, *n*-butyl, *iso*-butyl, *tert*-butyl, *n*-pentyl, *iso*-pentyl, *neo*-pentyl, *n*-hexyl, of which ethyl is the most preferred.

Where X represents a single bond or a group of formula -O-, $-C(=O)O-$, or $-NR_5C(=O)O-$, where $R_5$ is hydrogen, $C_1$-$C_4$ alkyl or $C_1$-$C_4$ acyl, the single bond and the groups -O- and $-NHC(=O)O-$ are most preferred.

Where q is 0 or 1 and t is (+) or means nothing, q=1 and t=(+) is most preferred.

Where $A^-$ represents a pharmaceutically acceptable anion this may be fluoride, chloride, bromide, iodide, methylsulfonate, *p*-toluenesulfonate, maleate, fumarate, nitrate, sulfate or oxalate, of which chloride, iodide, *p*-toluenesulfonate, methylsulfonate, and nitrate are more preferred, and iodide and chloride are most preferred.

The compounds of the present invention can exist in the form of various optical isomers and stereisomers because of the existence of asymmetric carbons in the molecule. The optical isomers can be resolved using conventional techniques of optical resolution to give optically active compounds. The diastereomers and geometrical isomers can be resolved using conventional techniques for separation such as recrystallization or chromatography. The present invention covers both the individual isomers and

mixtures (e.g. racemic mixtures) thereof, whether as obtained by the synthesis reaction or by mixing.

Examples of specific compounds of the invention are given in the following formulae, in which the number indicates the number of the example in which their preparation is described:

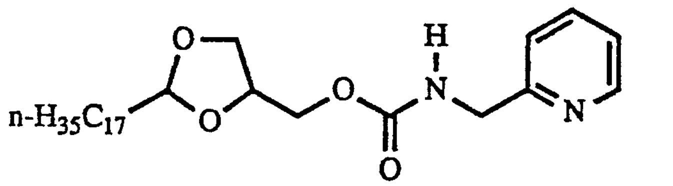

1

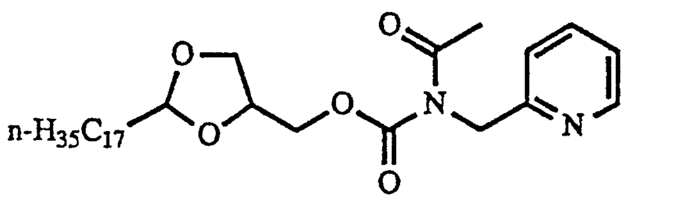

2

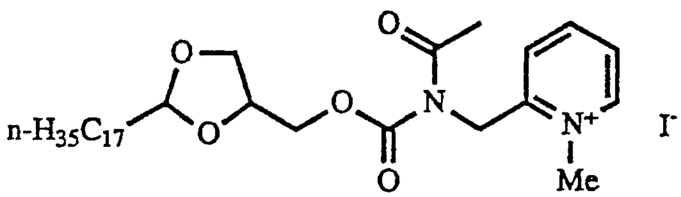

3

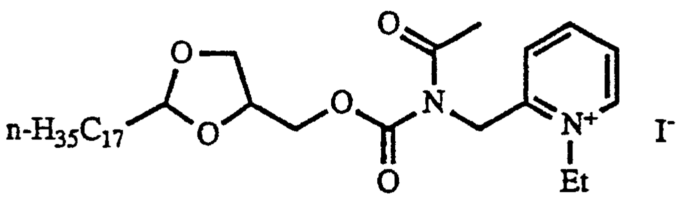

4

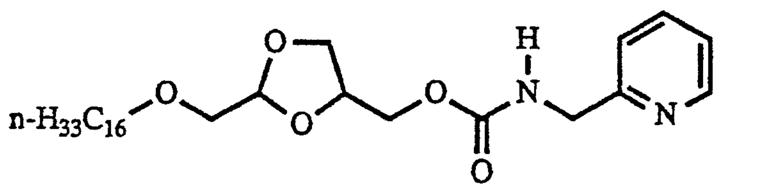

5

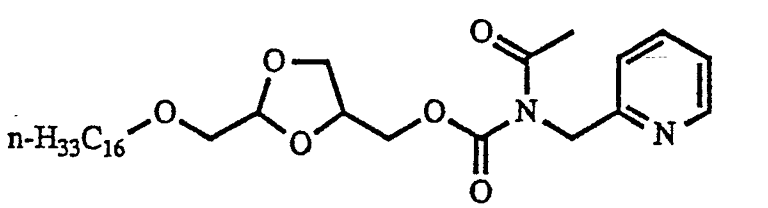

6

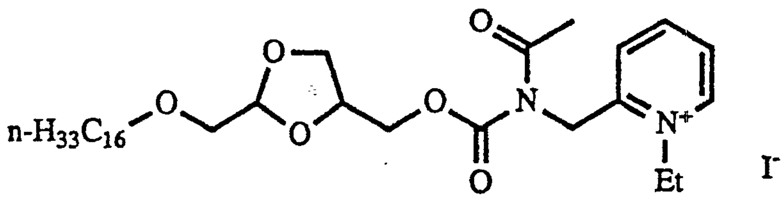

7

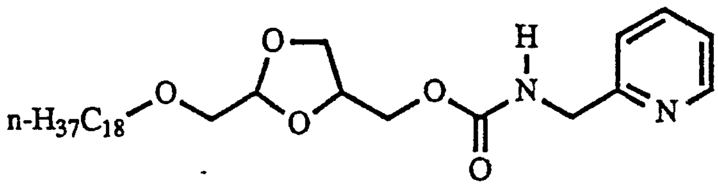

8

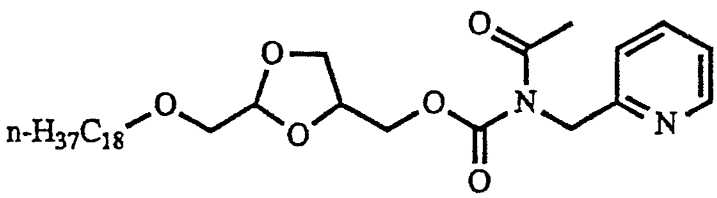

9

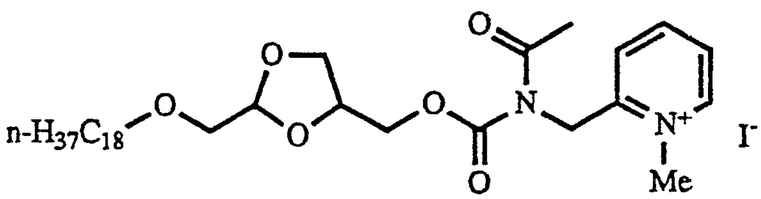

10

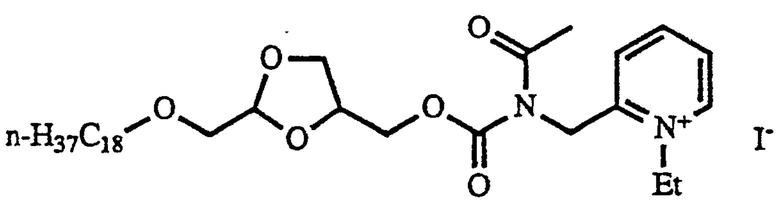

11

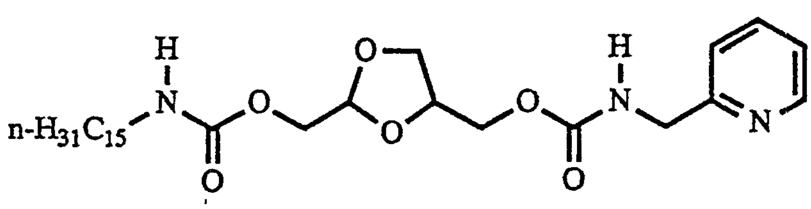

12

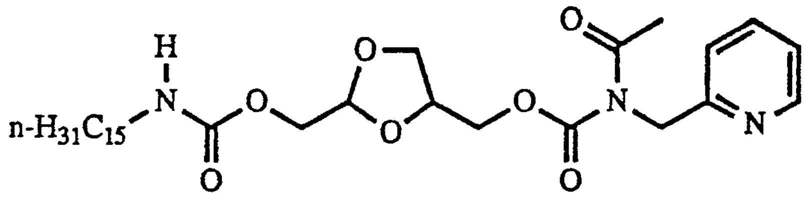

**13**

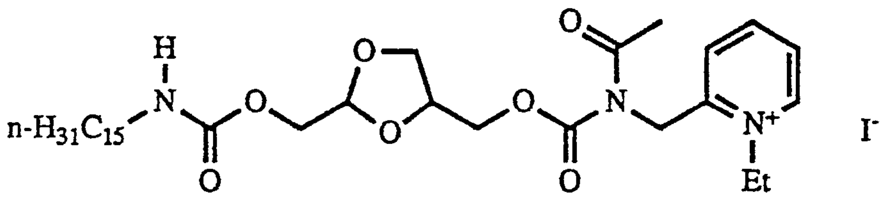

**14**

Of the compounds listed above, the following are most preferred:

No. 4: 2-(*N*-acetyl-*N*-(((2-heptadecyl-1,3-dioxolan-4-yl) methoxy) carbonyl) aminomethyl)-1-ethylpyridinium iodide.

No. 7: 2-(*N*-acetyl-*N*-(((2-hexadecyloxymethyl-1,3-dioxolan-4-yl) methoxy) carbonyl) aminomethyl)-1-ethylpyridinium iodide.

No. 11: 2-(*N*-acetyl-*N*-(((2-octadecyloxymethyl-1,3-dioxolan-4-yl) methoxy) carbonyl) aminomethyl)-1-ethylpyridinium iodide.

No. 14: 2-(N-acetyl-N-(((2-pentadecylcarbamoyloxymethyl-1,3-dioxolan-4-yl) methoxy) carbonyl) aminomethyl)-1-ethylpyridituum iodide.

The compounds of the present invention may be prepared by a variety of methods known for the preparation of this type of compounds. In general, the precise details of the method chosen for their preparation will vary depending on the nature of the compound. Scheme I shows the general strategy for their preparation According to that scheme, in the first step (step A) benzylglycerol (**II**) is allowed to react with aldehyde **III**, wherein $R_1$ and X have the previously described meaning. When X is a single bond, the aldehyde is obtained by oxidation of the corresponding fatty alcohol. When X is oxygen or aminocarbonyl, the aldehyde **III** is obtained by oxidative cleavage of the corresponding glycol. The reaction between compounds **II** and **III** takes place in the presence of a catalytic amount of an acid such as *p*-toluenesulfonic or camphorsulfonic acids. There is no particular restriction on the nature of the solvent to be employed, provided that it has no adverse effect on the reaction or on the reagents involved. We have found that toluene and/or benzene give good results with regard to yield and convenience to handle. The reaction can take place over a wide range of temperatures, and the precise reaction temperature is not critical to the invention. In general, we find it convenient to carry out the reaction at a temperature from 0 °C to that of the boiling solvent. If desired, the water produced in the reaction can be collected in a Dean-Stark condenser, although it is not strictly necessary. The time required for the reaction may also vary widely, depending on many factors, notably the reaction temperature and the nature of the reagents. However, provided that the raction is effected under the preferred conditions outlined above, a period of from 1 to 24 hours will usually suffice. Although the reaction is clean, if desired, the product **IV** can be purified by flash chromatography.

Step B consist in the deprotection of the hydroxyl function of compound **IV** to afford alcohol **V**. via hydrogenolysis at a hydrogen pressure that can range from 1 atm to 100 psi. There is no particular restriction on the nature of the solvent to be employed, provided that it has no adverse effect on the reaction or on the products involved. Examples of suitable solvents include: hydrocarbons, especially aromatic hydrocarbons, such as toluene; halogenated hydrocarbons, especially halogenated aliphatic hydrocarbons, such as dichloromethane and chloroform; ethers, such as tetrahydrofuran and diethyl ether; and alcohols, such as ethyl alcohol. The reaction is carried out in the presence of a metal catalyst. Commercially available 5% palladium on carbon is usually satisfactory. The time required for the reaction may also vary widely, depending on many factors, notably the reaction temperature and the hydrogen pressure At 50 psi, the reaction can be performed at room temperature, and a period of 15 hours will generally suffice. The product is isolated by filtration and concentration, and no purification is necessary.

In a second stage (step C), alcohol **V** is allowed to react with a compound **VI**, one of the so-called phosgene equivalents, that is to say, a doubly activated carbonyl compound. In compound **VI** the groups Y′

and Y″ are leaving groups and can be identical (Cl, imidazole, etc.) or different. Although in principle any phosgene equivalent described in the literature can be used, we have found that phenyl chlorocarbonate (**VI**, Y′ = Cl, Y″ = OPh) is an excellent reagent to carry out this reaction, due to its convenience to handle, and its low cost. The reaction is carried out in a

Scheme I

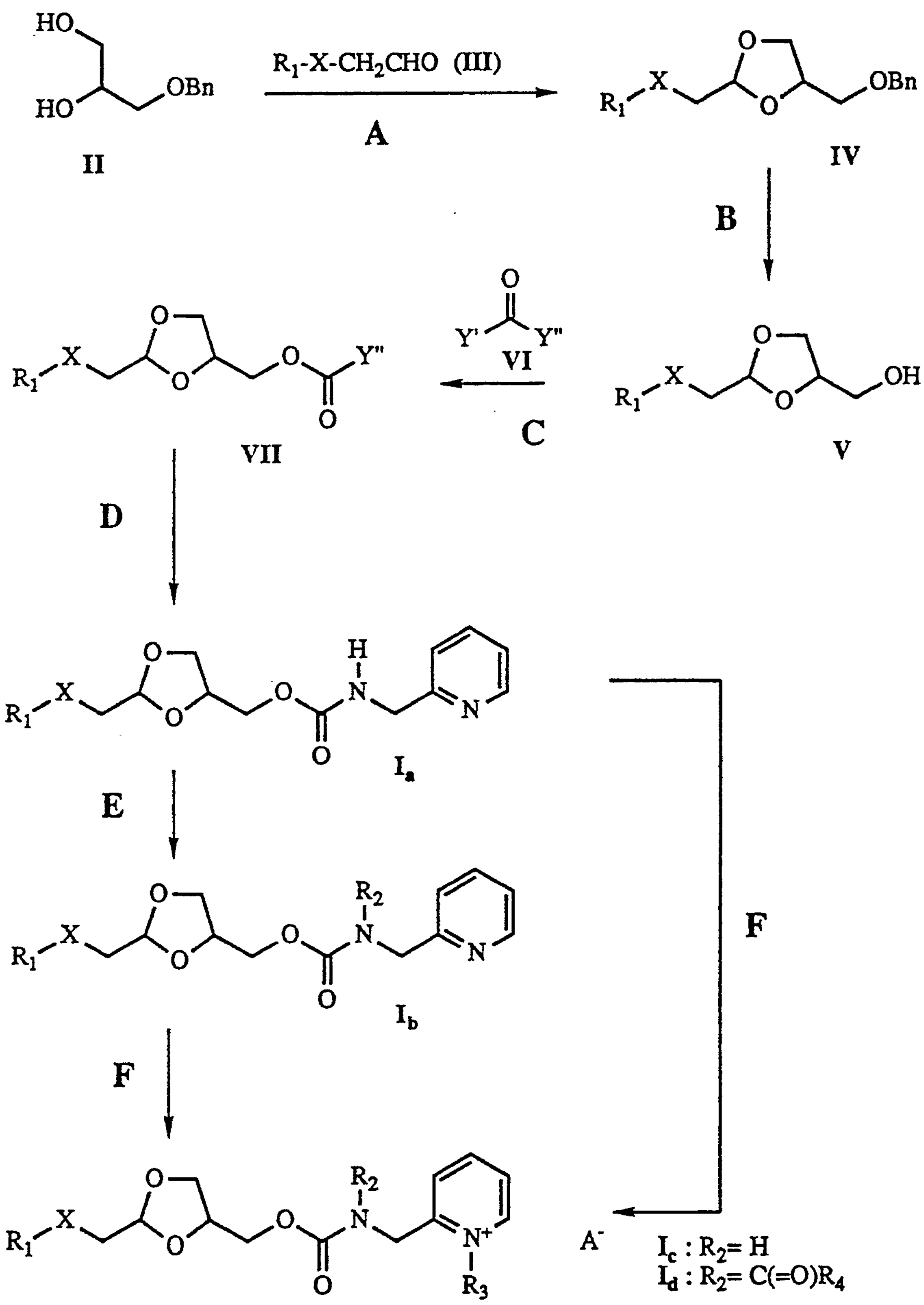

solvent in the presence of a proton scavenger base.There is no particular restriction on the nature of the solvent to be employed, provided that it has no adverse effect on the reaction or on the reagents involved. Examples of suitable solvents include: hydrocarbons, especially aromatic hydrocarbons, such as toluene; halogenated hydrocarbons, especially halogenated aliphatic hydrocarbons, such as dichloromethane and chloroform; and ethers, such as tetrahydrofuran and diethyl ether. Neither any particular restriction exists with respect to the base provided that it does not interfere with other parts of the molecule. It is preferred to use an amine, such as triethylamine or pyridine. The reaction can take place over a wide range of temperatures, and the precise reaction temperature is not critical to the invention. In general, we find it convenient to carry out the reaction at a temperetaure from 0 to 100° C, but temperatures from 0 to 50° C are preferred. The time required for the reaction may also vary widely, depending on many factors, notably the reaction temperature and the nature of the reagents. However, provided that the raction is effected under the preferred conditions outlined above, a period of from 30 min to 24 hours will usually suffice. The reaction is clean and usually purification of the product it is not necessary. However, if desired, product **VII** can be purified by flash chromatography.

In step D, compound **VII** (obtained in step C) is converted into the carbamate (**Ia**) by reaction with 2-picolylamine. The reaction can be performed in a solvent at a temperature and a period of time similar to those described in the step C. The crude reaction is washed, in this case, with an alkaline aqueous solution in order to remove the phenol produced during the reaction. The product obtained (**Ia**) can be purified by conventional methods such as flash chromatography.

Step E involves the derivatization of the carbamic nitrogen of compound (**Ia**) to give compound (**Ib**). The reaction involves the use of a reagent of formula $R_4C(=O)X$ where $R_4$ and X have the previous meaning. In the case in which $R_4$ is alkyl, a compound of formula $(R_4C(=O))_2O$ can also be used. There is no particular restriction on the nature of the solvent to be employed, provided that it has no adverse effect on the reaction or on the reagents involved. Examples of suitable solvents include halogenated hydrocarbons, especially halogenated aliphatic hydrocarbons, such as dichloromethane and chioroform. On the other hand, the reaction can be done in the presence of a proton scavenger amine, such as triethylamine. The use of the amine is optional since the pyridine ring has a basic nitrogen itself. The reaction can take place over a wide range of temperatures, and the precise reaction temperature is not critical to the invention. In general, we find it convenient to carry out the reaction at a temperetaure from 0° C to that of the boiling solvent, but a temperature near room temperature will generally suffice The time required for the reaction may also vary widely, depending on many factors, notably the reaction temperature and the nature of the reagents. However, provided that the raction is effected under the preferred conditions outlined above, a period of from 2 to 72 hours is usually enough. Once the reaction is complete, the desired product, of formula (**Ib**), can be isolated and purified by conventional methods such as flash chromatography.

Step F involves the transformation of a compound (**Ia**) or (**Ib**) into a compound or (**Ic**) or (**Id**), respectively. The reaction is carried out between the starting material and a reagent of formula $R_3A$ wherein $R_3$ is a lower alkyl group. The reaction can be performed without solvent in the case where $R_3A$ is liquid and non-volatile, or in the presence of a solvent when $R_3A$ is solid or very volatile, but in either case an excess of reagent is always used. The suitable solvents are preferentially those with high polarity. Examples of appropriate solvents include acetonitrile, tetrahydrofuran, dioxane, *N,N*-dimethylformamide and dimethylsulfoxide. The reaction can be performed between broad temperature margins and the precise temperature chosen is not particularly critical. We have found convenient to carry it out at a temperature between the room temperature and 120° C. The reaction time depends mainly on the nature of the reagent $R_3A$ and the temperature used, but a period between 1 hour and 72 hours will usually suffice. The desired compound can be isolated by concentration of the crude reaction mixture or precipitation with a less polar solvent. The compound obtained in this way is usually pure. However, if that is not the case, it can be purified by conventional techniques such as flash chromatography or recrystallization.

Compounds of formula (**Ic**) or (**Id**) are salts in which the anion $A^-$ comes from the reagent $R_3A$. If desired, such anion can be changed by using an ionic interchange resin or by selective salt precipitation..

Reflecting the activity of the present compounds, the invention further provides compositions which contain one or more of the compounds of the invention, together with a carrier and optionally other auxilliary agents, if necessary. The compounds of the present invention may be administered on the form of any conventional pharmaceutical formulation, the nature of which will, as is well known, depend on the route of administration and the nature of the condition to be treated. Thus, solid compositions according to the present invention for oral administration include compressed tablets, dispersible powders, granules and capsules. In tablets, one or more of the active component(s) is adrnixed with at least one inert diluent such as lactose, starch, mannitol, microcrystalline cellulose or calcium phosphate; granulating and desintegrating agents for example corn starch, gelatine, microcrystalline cellulose or polyvinylpyrrolidone; and lubricating

agents for example magnesium stearate, stearic acid or talc The tablets may be coated by known techniques to delay disintegration and absorption in the gastrointestinal tract and, thereby, provide a sustained action over a longer period. Gastric film-coated or enteric film-coated can be made with sugar, gelatin, hydroxypropylcellulose, or acrylic resins. Tablets with a sustained action may also be obtained using an excipient which provides regressive osmosis, such as the galacturonic acid polymers. Formulations for oral use may also be presented as hard capsules of absorbable material, such as gelatin, wherein the active ingredient is mixed with an inert solid diluent and lubricating agents, or pasty materials, such as etoxylated saturated glycerides. Soft gelatin capsules are possible wherein the active ingredient is mixed with water or an oil medium, for example peanut oil, liquid paraffin or olive oil.

Dispersible powders and granules suitable for preparation of a suspension by the addition of water provide the active ingredient in admixture with a dispersing or wetting agent, a suspending agent, such as sodium carboxymethylcellulose, sodium alginate, polyvinylpirrolidone, gum tragacanth, xantham gum, gum acacia, and one or more preservatives, such as methyl or *n*-propyl-*p*-hydroxybenzoate. Additional excipients, for example sweetening, flavoring and coloring agents may also be present.

Liquid compositions for oral administration include emulsions, solutions, suspensions, syrups and elixirs containing commonly used inert diluents, such as distilled water, ethanol, sorbitol, glycerol, or propylene glycol. Such compositions may also comprise adjuvants such as wetting agents, suspending agents, sweetening, flavoring, perfuming, preserving agents and buffers.

Other compositions for oral administration include spray compositions, which may be prepared by known methods and which comprise one or more active compound(s). The spray compositions will contain a suitable propellent.

Preparations for injection according to the present invention for parenteral administration include sterile aqueous or non-aqueous solutions, suspensions or emulsions, in a non-toxic parentally-acceptable diluent or solvent. Examples of aqueous solvents or suspending media are distilled water for injection, the Ringer's solution, and isotonic sodium chloride solution. Examples of non-aqueous solvents or suspending media are propylene glycol, polyethylene glycol, vegetable oils such as olive oil, or alcohols such as ethanol. These compositions may also include adjuvants such as wetting, preserving, emulsifying and dispersing agents. They may be sterilized by one of the known methods or manufactured in the form of sterile solid compositions which can be dissolved in sterile water or some other sterile injectable medium immediately before use. When all of the components are sterile, the injectables will maintain the sterility if they are manufactured in sterile environment.

A compound of the invention may also be administered in the form of suppositories for rectal administration of the drug, or as creams, ointments jellies, solutions or suspensions for topical use and pessaries for vaginal administration.

The dosage and frequency of dose may vary depending upon symptoms, age and body weight of the patient, as well as upon the route of administration, but, in general, the compounds of the invention may be administered orally in a daily dose of from 5 to 2,000 mg for an adult, preferably a dosage of from 25 to 500 mg, which may be administered either as a single dose or as divided doses.

The invention is further illustrated by the following examples, which describe the preparation of various of the compounds of the invention, as well as the preparations of various of the starting materials which may be used in the Examples for the preparation of the compounds of the present invention.

## PREPARATION 1

### 2-Heptadecyl-4-benzyloxymethyl-1,3-dioxolane.

A mixture of benzylglycerol (1.48 g, 8.12 mmol), octadecanal (2.40 g, 8.9 mmol) and camphorsulfonic acid (189 mg, 0.81 mmol) in toluene (20 mL) was stirred 20 hours at room temperature. Then, an aqueous solution of phosphate buffer (10 mL) was added to the mixture, and the organic layer was separated, washed with brine and dried over anhydrous sodium sulfate. The organic solution was filtered and concentrated to give 4 g of a white solid which was purified by flash chromatography (ethyl acetate:hexane 1:20) yielding the title compound as a white solid (2.74 g, 78%).

mp: 36-38 °C;

IR (KBr) $\nu_{max}$ ($cm^{-1}$):

3027, 2921, 2849, 1492, 1462, 1406, 1372, 1339, 1309, 1126, 1028, 957, 735, 697;

$^1H$ NMR (80 MHz, $CDCl_3$) δ (TMS):
7.31 (s, 5H, Ar),
4.88 (t J=4.5Hz, 1H, OCHO),
4.56 (s,2H, Ar-$CH_2$),
4.3-4.0 (m, 1H),
4.0-3.7 (m, 2H),
3.7-3.3 (m, 2H),
2.0-0.7 (m, 35H aprox.).

## PREPARATION 2.

2-Heptadecyl-1,3-dioxolane-4-methanol.

A mixture of the product obtained in the preparation 1 (2.56 g, 5.91 mmol) in dichloromethane (35 mL) and ethanol (5 mL) together with a catalytic amount of 5% palladium on carbon (400 mg) was hydrogenated at 100 psi during 18 hours at room temperature. The mixture was filtered and concentrated *in vacuo* to yield the title compound as a white solid (2.00 g, 99%).
mp: 50-51 °C;
IR (KBr) $\nu_{max}$ ($cm^{-1}$):
3240, 2916, 2846, 1464, 1129, 1043, 958, 721;
$^1H$ NMR (80 MHz, $CDCl_3$) δ (TMS):
4.89 (t, J=4.5Hz, 1H, OCHO),
4.3-4.0 (m, 1H),
3.84(dd,J=1.8Hz, J=6Hz, 2H),
3.7-3.4 (m, 2H),
2.09(s, 1H, OH),
1.7-0.7 (m, 35H aprox.).
*Analysis* calcd. for $C_{21}H_{42}O_3$ : C 73.62 %; H 12.36%.
Found: C 73.83%; H 12.58%.

## PREPARATION 3

Hexadecyloxyethanal, hydrate.

To a solution of sodium periodate (3 g, 14 mmol) in water was added a solution of hexadecylglycerol (2.27 g, 7.17 mmol) in acetone (100 mL), resulting in a gradual precipitation of a white solid. After 90 minutes, cold water was added (1.5 L), and the mixture was stirred 1 hour, filtered and dried to afford the title compound as a white solid (2.11 g, 100%).
mp: 56-58 °C;
IR (KBr) $\nu_{max}$ ($cm^{-1}$):
3397, 2913, 2845, 1464, 1134, 890;
$^1H$ NMR (80 MHz, $CDCl_3$) δ (TMS):
3.6-3.4 (m, 4H, $CH_2OCH_2$),
1.7-0.7 (m, 31H aprox.).

## PREPARATION 4

Octadecyloxyethanal, hydrate.

Following the procedure described in preparation 3, but using octadecylglycerol instead of hexadecylglycerol, the title compound was obtained in a similar yield.
mp: 62-63 °C;
IR (KBr) $\nu_{max}$ ($cm^{-1}$): 3419, 2914, 2846, 1464, 1136;
$^1H$ NMR (80 MHz, $CDCl_3$) $\delta$ (TMS):
9.80(t, J=1Hz, 1/10H, CHO),
4.01(d, J=1Hz, 2/10H, $CH_2CHO$),
3.5-3.3 (m, $CH_2OCH_2CH(\overline{OH})_2$),
1.7-0.7 (m, 35H aprox.).

## PREPARATION 5

2-Hexadecyloxymethyl-4-benzyloxymethyl-1,3-dioxolane.

Following the procedure described in preparation 1, but using the product obtained in preparation 3 instead of octadecanal, the title compound was obtained as a colorless oil in a similar yield.
IR (film) $\nu_{max}$ ($cm^{-1}$):
3058, 3025, 2918, 2849, 1491, 1464, 1362, 1118, 1028, 734, 697;
$^1H$ NMR (80 MHz, $CDCl_3$) $\delta$ (TMS):
7.31(s, 5H, Ar),
5.04(t,J=4Hz, 1H, OCHO),
4.55(s, 2H, Ar-$CH_2$),
4.26(quint, J=5.5Hz, 1H),
4.1-3.8 (m, 2H),
3.7-3.3 (m, 6H),
1.7-0.7 (m, 31H aprox.).

## PREPARATION 6

2-Octadecyloxymethyl-4-benzyloxymethyl-1,3-dioxolane.

Following the procedure described in preparation 1, but using the product obtained in preparation 4, instead of octadecanal, the title compound was obtained as a colorless oil in a similar yield.
IR (film) $\nu_{max}$ ($cm^{-1}$):
3058, 3024, 2918, 2848, 1490, 1466, 1363, 1118, 1030, 735, 698;
$^1H$ NMR (80 MHz, $CDCl_3$) $\delta$ (TMS):
7.30 (s, 5H, Ar),
5.03 (t, J=4Hz, 1H, OCHO),
4.55 (s, 2H, Ar-$CH_2$), 4.26 (quint, J=5.5Hz, 1H),
4.1-3.8 (m, 2H),
3.7-3.3 (m, 6H),
1.7-0.7 (m, 31H aprox.).

## PREPARATION 7

2-Hexadecyloxymethyl-1,3-dioxolane-4-methanol.

Following the procedure described in preparation 2, but using the compound obtained in preparation 5 instead of that of preparation 1, the title compound was obtained as a white solid in a similar yield. mp: 34-35 °C;
IR (KBr) $\nu_{max}$ ($cm^{-1}$):
3381, 2912, 2845, 1464, 1375, 1238, 1128, 867, 721, 558, 488;
$^1H$ NMR (80 MHz, $CDCl_3$) $\delta$ (TMS):
5.09 (t, J=2.5Hz, 1H, OCHO),
4.3-3.7 (m, 3H, $OCH_2CHO$),
3.7-3.4 (m, 6H, $CH_2OH$, $CH_2OCH_2$),
2.4 (broad s, 1H, OH),
1.7-0.7 (m, 31H aprox.).
*Analysis* calcd. for $C_{21}H_{42}O_4$ : C 70.35 %; H 11.81%
Found: C 70.02%; H 12.09%.

## PREPARATION 8

2-Octadecyloxymethyl-1,3-dioxolane-4-methanol.

Following the procedure described in preparation 2, but using the compound obtained in preparation 6 instead of that of preparation 1, the title compound was obtained in a similar yield.
mp: 37-39 °C;
IR (KBr) $\nu_{max}$ ($cm^{-1}$):
3422, 2914, 2846, 1464, 1375, 1245, 1130, 1042, 863, 721, 557; $^1H$ NMR (80 MHz, $CDCl_3$) $\delta$ (TMS):
5.08 (t, J=2.5Hz, 1H, OCHO),
4.3-3.7 (m, 3H, $OCH_2CHO$),
3.7-3.4 (m, 6H, $CH_2OH$, $CH_2OCH_2$),
3.0 (m, 1H, OH),
1.7-0.7 (m, 35H aprox.).

## PREPARATION 9

2,2-Dimethyl-4-phenoxycarbonyloxymethyl-1,3-dioxolane.

To a cooled solution (0 °C) of solketal (13.21 g, 0.1 mol) and pyridine (16 mL, 0.2 mol) in dichloromethane (250 mL) was slowly added phenyl chlorocarbonate (14.47 mL, 0.115 mol). The mixture was stirred 1 hour at 25 °C. Then, dichloromethane (100 mL) was added, and the solution was washed successively with an aqueous solution of 2N HCl (2x100 mL), 10% aqueous sodium bicarbonate (1x100 mL) and brine (1x100 mL). The organic phase was dried over anhydrous sodium sulfate and filtered. The filtrate was concentrated *in vacuo* to afford the title compound as a yellowish dense liquid (29.9 g).
IR (film) $\nu_{max}$ ($cm^{-1}$):
3041, 2983, 2933, 1758, 1589, 1488, 1451, 1380, 1369, 1238, 1208, 1180, 1158, 1061, 1022, 839, 776;

## PREPARATION 10

2-Benzyloxymethyl-4-phenoxycarbonyloxymethyl-1,3-dioxolane.

Following the procedure described in preparation 1, but using the product obtained in preparation 9 and benzyloxyethanal, instead of benzylglycerol and octadecanal, respectively, the title compound was obtained as a colorless dense liquid, in a similar yield.
IR (film) $\nu_{max}$ ($cm^{-1}$):
3058, 3026, 2912, 1759, 1588, 1489, 1448, 1385, 1336, 1243, 1208, 1154, 1110, 774, 738, 698;

$^1H$ NMR (80 MHz, $CDCl_3$) δ (TMS):
7.4-7.5 (m, 10H, Ar),
5.14 (t, J=4Hz, 1H, OCHO),
4.61 (s, 2H, Ar-$CH_2$),
4.5-3.6 (m, 5H),
3.7-3.4 (m,2H).

## PREPARATION 11

4-Phenoxycarbonyloxymethyl-1,3-dioxolane-2-methanol.

Following the procedure described in preparation 2, but using the compound obtained in preparation 10, instead of that of preparation 1, the title compound was obtained as a colorless liquid in a similar yield.
IR (film) $\nu_{max}$ ($cm^{-1}$):
3430, 2879, 1758, 1588, 1485, 1452, 1385, 1340, 1247, 1208, 1146, 1060, 1022, 775;
$^1H$ NMR (80 MHz, $CDCl_3$) δ (TMs):
7.5-7.0 (m, 5H, Ar),
5.03 (t, J=3Hz, 1H, OCHO),
4.7-4.2 (m, 3H),
4.2-3.8 (m, 1H),
3.8-3.5 (m, 2H),
2.68 (s, 1H, OH),.

## PREPARATION 12

2-Pentadecylcarbamoyloxymethyl-4-phenoxycarbonyloxymethyl-1,3-dioxolane.

A solution of the compound obtained in preparation 13 (1.61 g, 6.33 mmol), pentadecyl isocyanate (2.40 g, 9.5 mmol), pyridine (5.1 mL) and chloroform (10 mL) was heated to reflux during 6 hours. Then, a 6N HCl aqueous solution was added. The organic layer was separated, dried over anhydrous sodium sulfate and filtered. The filtrate was concentrated *in vacuo* to give a paste (9.5 g) that solidified on standing.
mp: 38-40 °C;
IR (KBr) $\nu_{max}$ ($cm^{-1}$):
3302, 2917, 2848, 1759, 1714, 1632, 1588, 1525, 1244, 1209, 1139, 1060, 775, 721;
$^1H$ NMR (80 MHz, $CDCl_3$) δ (TMS):
7.5-7.0 (m, 5H, Ar),
5.16 (t, 7=3.5Hz, 1H, OCHO),
4.7-3.5 (m, 7H),
3.14 (q, J=6Hz, 2H, $CH_2N$),
1.7-0.7 (m, 29H aprox).

## EXAMPLE 1

2-(*N*-(((2-Heptadecyl-1,3-dioxolan-4-yl) methoxy) carbonyl) aminomethyl)pyridine

A flame dried flask was charged with the compound obtained in preparation 2 (2.84 g, 7.92 mmol), dry dichloromethane (25 mL) and dry pyridine (1.27 mL, 15.8 mmol). The solution was cooled at 0 °C and phenyl chlorocarbonate (1.09 mL, 8.71 mmol) was slowly added. The mixture was stirred 1 h at 25 °C, then, dichloromethane was added (40 mL) and the solution was washed with 1N aqueous HCl solution. The

solution was dried over anhydrous sodium sulfate, filtered and concentrated to afford the corresponding mixed carbonate (3.91 g). This substance (3.88 g, 8.10 mmol) was solved in dry chloroform (15 mL) and then, 2-picolylamine (1.05 mL, 9.73 mmol) was added. The reddish solution was stirred 18 hours at reflux. Then, dichloromethane was added (40 mL) and the solution washed with 1N aqueous NaOH solution (3x30 mL), followed by brine. The organic layer was dried over anhydrous sodium sulfate, filtered and concentrated to afford the desired product as a brown semi-solid (4.14 g). The reaction crude was purified by flash chromatography (ethyl acetate:hexane 3:1) to yield the title compound as a white solid (3.76 g, 96% overall yield, two steps).

mp: 108-111 °C;
IR (KBr) $\nu_{max}$ ($cm^{-1}$):
3302, 3056, 2915, 2845, 1673, 1624, 1587, 1566, 1540, 1466, 1302, 1262, 1225, 1161, 1133, 1055, 956;
$^1H$ NMR (60 MHz, $CDCl_3$) $\delta$ (TMS):
8.7 (m, 1H, pyr),
7.7 (t, J=7Hz, 1H, pyr),
7.3-7.0 (m, 2H, pyr),
6.0 (broad s., 1H, NH),
5.1-4.9 (m, 1H, OCHO),
4.5 (d, J=6Hz, 2H, pyr-$CH_2$),
4.6-3.5 (m, 5H),
1.7-0.7 (m, 35H aprox.).

## EXAMPLE 2

2-(*N*-Acetyl-*N*-(((2-heptadecyl-1,3-dioxolane-4-yl)methoxy)carbonyl)aminomethyl) pyridine.

In a dry flask, the compound obtained in example 1 (1.5 g, 3.04 mmol), was dissolved in dry dichloromethane (10 mL). The solution was cooled at 0 °C and acetyl chloride (0.28 mL, 3.96 mmol) was slowly added. The reaction mixture was stirred 1.5 h at 0 °C and 48 h at room temperature. Then, triethylamine was added, the mixture was diluted with dichloromethane and washed with water (2 x 40 mL). The organic layer was dried over anhydrous sodium sulfate, filtered and concentrated to afford a brown solid (2.12 g) which was purified by flash chromatography (ethyl acetate: hexane 1:1), yielding the desired compound (1.54 g, 95%).

mp: 57-59 °C;
IR (KBr) $\nu_{max}$ ($cm^{-1}$):
2914, 2845, 1732, 1701, 1590, 1567, 1464, 1429, 1385, 1369, 1354, 1295, 1204, 1157, 1124;
$^1H$ NMR (60 MHz, $CDCl_3$) $\delta$ (TMS):
8.7 (m, 1H, pyr),
7.7 (t, J=7Hz, 1H, pyr),
7.3-7.0 (m, 2H, pyr),
5.1 (s, 2H, pyr-$CH_2$),
5.0-4.9 (m, 1H, OCHO),
4.4-3.5 (m, 5H),
2.6 (s, 3H, O=$CCH_3$),
1.7-0.7 (m, 35H aprox.).
*Analysis* calcd. for $C_{30}H_{50}N_2O_5$: C 69.46%; H 9.72%; N 5.40%.
Found: C 69.53%; H 10.20%; N 5.41%.

## EXAMPLE 3

2-(*N*-Acetyl-*N*-(((2-heptadecyl-1,3-dioxolane-4-yl)methoxy)carbonyl)aminomethyl)-1-methylpyridinium iodide.

A solution of the compound obtained in example 2 (200 mg, 0.385 mmol) in acetonitrile (0.5 mL) and

methyl iodide (0.5 mL) was heated three days at 70° C. The solution was concentrated *in vacuo*, the residue was dissolved in dichloromethane and precipitated with ether. The solid was filtered and dried to yield the desired compound as a yellowish powder (204 mg, 79%).
mp:76-84° C;
IR (KBr) $\nu_{max}$ ($cm^{-1}$):
3447 ($H_2O$), 2914, 2846, 1730, 1680, 1626, 1580, 1509, 1463, 1367, 1210, 988.
*Analysis* calcd. for $C_{31}H_{53}IN_2O_5$ •$H_2O$: C 54.86%; H 8.17%; N 4.13%.
Found: C 55.15%; H 8.39%; N 4.42%.

## EXAMPLE 4

2-(*N*-acetyl-*N*-(((2-heptadecyl-1,3-dioxolane-4-yl)methoxy)carbonyl)aminomethyl)-1-ethylpyridinium iodide.

Following the procedure described in example 3, but using ethyl iodide instead of methyl iodide, the title compound was obtained in a similar yield.
mp: 77-80° C;
IR (KBr) $\nu_{max}$ ($cm^{-1}$):
3491 ($H_2O$), 3033, 2949, 2918, 2848, 1757, 1685, 1623, 1575, 1509, 1483, 1464, 1367, 1295, 1277, 1210, 1123, 1079;
$^1H$ NMR (80 MHz, $CDCl_3$) $\delta$ (TMS):
9.61 (broad d, J=6Hz, 1H, pyr),
8.43 (dt, $J_d$=1.8Hz, $J_t$=7.5Hz, 1H, pyr),
8.03 (broad t, J=6Hz, 1H, pyr),
7.78 (broad d, J=8Hz, 1H, pyr),
5.46 (s, 2H, pyr-$CH_2$),
5.05 (q, J=7Hz, 2H, NEt),
4.9-4.6 (m, 1H),
4.4-4.2 (m, 3H),
4.0-3.7 (m, 1H),
2.67 (s, 3H, O=$CCH_3$),
1.74 (t, J=7Hz, 3H, NEt),
1.7-0.7 (m, 35H aprox.)
*Analysis* calcd. for $C_{32}H_{53}IN_2O_5$ •$H_2O$: C 55.68%; H 8.03%; N 4.06%.
Found: C 55.18%; H 8.37%; N 4.05%.

## EXAMPLE 5

2(*N*-(((2-Hexadecyloxymethyl-1,3-dioxolan-4-yl)methoxy)carbonyl)aminomethyl) pyridine.

Following the procedure described in example 1, but using the product obtained in preparation 7, instead of the one prepared in preparation 2, the title compound was obtained as a white solid in a similar yield.
mp: 75-76° C;
IR (KBr) $\nu_{max}$ ($cm^{-1}$):
3214, 2918, 2844, 1706, 1592, 1567, 1536, 1462, 1277, 1124;
$^1H$ NMR (80 MHz, $CDCl_3$) $\delta$ (TMS):
8.55 (dq, $J_d$=1Hz, $J_q$=6Hz, 1H, pyr.),
7.7 (dt, $J_d$=1.8Hz, $J_t$=7.5Hz, 1H, pyr),
7.3-7.0 (m, 2H, pyr),
5.9 (broad s, 1H, NH),
5.07 (t, J=4.5Hz, 1H, OCHO),
4.50 (d, J=5.4Hz, 2H, pyr-$CH_2$),

4.4-4.1 (m, 3H),
3.89 (t, J=6Hz, 1H),
3.6-3.4 (m, 4H, $CH_2OCH_2$),
1.7-0.7 (m, 31H aprox.).
*Analysis* calcd. for $C_{28}H_{48}N_2O_5$: C 68.26%; H 9.82%; N 5.69%.
Found: C 68.32%; H 10.18%; N 5.99%.

## EXAMPLE 6

2-(*N*-Acetyl-*N*-(((2-hexadecyloxymethyl-1,3-dioxolan-4-yl)methoxy)carbonyl) aminomethyl) pyridine.

Following the procedure described in example 2, but using the product prepared in example 5, instead of the one prepared in example 1, the title compound was obtained as a white solid in a similar yield.
mp: 45-46° C;
IR (KBr) $\nu_{max}$ ($cm^{-1}$):
2913, 2844, 1731, 1699, 1589, 1567, 1464, 1440, 1367, 1354, 1294, 1204, 1151, 1126, 1068, 972, 958;
$^1H$ NMR (80 MHz, $CDCl_3$) $\delta$ (TMS):
8.50 (dq, $J_d$=5Hz, $J_q$=1Hz, 1H, pyr.),
7.61 (dt, $J_d$=1.8Hz, $J_t$=7.5Hz, 1H, pyr),
7.2-7.0 (m, 2H, pyr),
5.08 (s, 2H, pyr-$CH_2$), 4.97 (t, J=4Hz, 1H, OCHO),
4.3-4.1 (m, 3H),
3.9-3.6 (m, 2H),
3.6-3.3 (m, 4H, $CH_2OCH_2$),
2.62 (s, 3H, O=$CCH_3$),
1.7-0.7 (m, 31H aprox.).
*Analysis* calcd. for $C_{30}H_{48}N_2O_6$: C 67.64%; H 9.08%; N 5.26%
Found: C 67.42%; H 9.09%; N 5.07%.

## EXAMPLE 7

2-(*N*-Acetyl-*N*-(((2-hexadecyloxymethyl-1,3-dioxolan-4-yl)methoxy)carbonyl) aminomethyl)-1-ethylpyridinium iodide.

Following the procedure described in example 3, but using the product prepared in example 6 and ethyl iodide, instead of the one prepared in example 2 and methyl iodide, the title compound was obtained as a yellow solid in a similar yield.
mp: 63-66° C;
IR (KBr) $\nu_{max}$ ($cm^{-1}$):
3032 ($H_2O$), 2917, 2848, 1758, 1684, 1623, 1575, 1507, 1480, 1465, 1422, 1366, 1280, 1215, 1165, 1121, 1105, 1080;
$^1H$ NMR (80 MHz, $CDCl_3$) $\delta$ (TMS):
9.56 (broad d, J=6Hz, 1H, pyr),
8.58 (t, J=7.5Hz, 1H, pyr),
8.08 (t, J=6.3Hz, 1H, pyr),
7.95 (d, J=8Hz, 1H, pyr),
5.48 (s, 2H, pyr-$CH_2$),
5.05 (q, J=7Hz,2H,NEt),
5.00 (t, 1H, OCHO),
4.6-4.3 (m, 3H),
4.3-3.7 (m, 2H),
3.7-3.3 (m, 4H, $CH_2OCH_2$),

2.67 (s, 3H, O=CCH$_3$),
1.73 (t, J=7Hz, 3H, NEt),
1.7-0.7 (m, 31H aprox.)
*Analysis* calcd. for $C_{32}H_{55}IN_2O_6$ •1/2$H_2O$: C 54.93%; H 8.07%; N 4.00%.
Found: C 54.92%; H 7.95%; N 3.86%.

## EXAMPLE 8

2-(*N*-(((2-Octadecyloxymethyl-1,3-dioxolan-4-yl)methoxy)carbonyl)aminomethyl) pyridine.

Following the procedure described in example 1, but using the product prepared in preparation 8, instead of the one obtained in preparation 2, the title compound was obtained as a white solid in a similar yield.
mp: 41-45 °C;
IR (KBr) $\nu_{max}$ (cm$^{-1}$):
3198, 2923, 2844, 1758, 1706, 1592, 1567, 1531, 1462, 1435, 1277, 1251, 1126;
$^1$H NMR (80 MHz, CDCl$_3$) δ (TMS):
8.50 (broad d, J=5.5Hz, 1H, pyr.),
7.7 (dt, $J_d$=1.8Hz, $J_t$=7.5Hz, 1H, pyr),
7.3-7.0 (m, 2H, pyr),
5.8 (broad s, 1H, NH),
5.03 (t, J=4.5Hz, 1H, OCHO),
4.49 (d, J=5.5Hz, 2H, pyr-CH$_2$),
4.4-4.1 (m, 3H),
3.89 (t, J=6Hz, 1H),
3.6-3.4 (m, 4H, CH$_2$OCH$_2$),
1.7-0.7 (m, 35H aprox.).
*Analysis* calcd. for $C_{30}H_{52}N_2O_5$ : C 69.19%; H 10.07%; N 5.38%.
Found: C 68.83%; H 9.74%; N 6.09%.

## EXAMPLE 9

2-(*N*-Acetyl-*N*-(((2-octadecyloxymethyl-1,3-dioxolan-4-yl)methoxy)carbonyl)aminomethyl)pyridine.

Following the procedure described in example 2, but using the product prepared in example 8, instead of the one prepared in example 1, the title compound was obtained as a white solid, in a similar yield.
mp: 51-52 °C;
IR (KBr) $\nu_{max}$ (cm$^{-1}$):
2914, 2845, 1730, 1700, 1589, 1567, 1440, 1368, 1354, 1294, 1205, 1151, 1127, 972;
$^1$H NMR (80 MHz, CDCl$_3$) δ (TMS):
8.49 (broad d, J=5Hz, 1H, pyr.),
7.60 (dt, $J_d$=1.8Hz, $J_t$=7.5Hz, 1H, pyr),
7.2-7.0 (m, 2H, pyr),
5.08 (s, 2H, pyr-CH$_2$),
4.98 (t, J=4Hz, 1H, OCHO),
4.3-4.1 (m, 3H),
3.9-3.6 (m, 2H),
3.6-3.3 (m, 4H, CH$_2$OCH$_2$),
2.62 (s, 3H, O=CCH$_3$),
1.7-0.7 (m, 35H aprox.).
*Analysis* calcd. for $C_{32}H_{54}N_2O_6$ : C 68.29%; H 9.67%; N 4.98%.
Found: C 68.41%; H 9.86%; N 4.99%.

## EXAMPLE 10

2-(*N*-Acetyl-*N*-(((2-octadecyloxymethyl-1,3-dioxolan-4-yl)methoxy)carbonyl)aminomethyl)-1-methylpyridinium iodide.

Following the procedure described in example 3, but using the product prepared in example 9, instead of the one prepared in example 2, the title compound was obtained as a yellow solid in a similar yield.
mp: 51-65 °C;
IR (KBr) $\nu_{max}$ ($cm^{-1}$):
3439 ($H_2O$), 2913, 2846, 1747, 1679, 1626, 1580, 1510, 1463, 1367, 1213, 1122;
$^1H$ NMR (80 MHz, $CDCl_3$) $\delta$ (TMS):
9.43 (broad d, J = 6.5Hz, 1H, pyr.),
8.46 (broad t, J = 7.5Hz, 1H, pyr),
7.98 (broad t, J = 6.5Hz, 1H, pyr),
7.85 (broad d, J = 8Hz, 1H, pyr),
5.45 (s, 2H, $NCH_2$),
4.99 (t, J = 3.7Hz, 1H, OCHO),
4.68 (s, 3H, NMe),
4.5-4.3 (m, 3H),
4.0-3.7 (m, 1H),
3.6-3.3 (m, 4H, $CH_2OCH_2$),
2.66 (s, 3H, $OCCH_3$),
1.7-0.7 (m, 35H aprox.).
*Analysis* calcd. for $C_{33}H_{37}IN_2O_6 \bullet H_2O$: C 54.84%; H 8.23%; N 3.88%.
Found: C 54.84%; H 8.06%; N 3.88%.

## EXAMPLE 11

2(*N*-Acetyl-*N*-(((2-octadecyloxymethyl-1,3-dioxolan-4-yl)methoxy)carbonyl)aminomethyl)-1-ethylpyridinium iodide.

Following the procedure described in example 3, but using the product prepared in example 9 and ethyl iodide, instead of the one prepared in example 2 and methyl iodide, the title compound was obtained as a yellow solid in a similar yield.
mp: 47-50 °C;
IR (KBr) $\nu_{max}$ ($cm^{-1}$):
3443 ($H_2O$), 2913, 2846, 1747, 1684, 1625, 1579, 1463, 1367, 1210, 1162, 1122, 1088;
$^1H$ NMR (80 MHz, $CDCl_3$) $\delta$ (TMS):
9.17 (broad d, J = 6Hz, 1H, pyr.),
8.57 (dt, $J_d$ = 1.8Hz, $J_t$ = 7.5Hz, 1H, pyr),
8.03 (t, J = 8Hz, 2H, pyr),
5.37 (s, 2H, pyr-$CH_2$),
4.91 (t, J = 4Hz, 1H, OCHO),
4.75 (q, J = 7Hz, 2H, NEt), 4.5-4.1 (m, 3H),
3.9-3.6 (m, 1H),
3.6-3.3 (m, 4H, $CH_2OCH_2$),
2.63 (s, 3H, O = $CCH_3$),
1.57 (t, J = 7HZ, 3H, NEt),
1.7-0.7 (m, 35H aprox.).
*Analysis* calcd. for $C_{34}H_{59}IN_2O_6 \bullet 1/2H_2O$: C 56.12%; H 8.31%; N 3.85%.
Found: C 55.84%; H 8.30%; N 3.84%.

EXAMPLE 12

2-(*N*-(((2-Pentadecylcarbamoyloxymethyl-1,3-dioxolan-4-yl)methoxy)carbonyl) aminomethyl)pyridine.

A solution of the product obtained in preparation 12 (4 g, 7.88 mmol), 2-picolylamine (1.02 g, 9.45 mmol) and chloroform (30 mL) was heated to reflux. The resulting reddish solution was kept at reflux during 18 hours. Then, dichloromethane (40 mL) was added and the solution was washed with 1N aqueous NaOH (3x30 mL), followed by brine. The organic phase was dried over anhydrous sodium sulfate, filtered and concentrated to give the desired compound as a slightly brown semisolid (5 g). The crude reaction mixture was purified by flash chromatography (ethyl acetate) to yield the title compound as a white solid (2.20 g, 53%).

mp: 71-72 °C;

IR (KBr) $\nu_{max}$ ($cm^{-1}$):

3315, 3226, 3043, 2914, 2846, 1700, 1686, 1591, 1568, 1530, 1464, 1433, 1269, 1253, 1143, 1093, 1049';

$^1$H NMR (80 MHz, $CDCl_3$) δ (TMS):

8.54 (broad d, J=5Hz, 1H, pyr.),

7.68 (dt, $J_d$=1.8Hz, $J_t$=7.5Hz, 1H, pyr),

7.3-7.0 (m, 2H, pyr),

5.9 (broad s, 1H, NH),

5.13 (t, J=3.8Hz, 1H, OCHO),

4.7 (broad s, 1H, NH),

4.49 (d, J=5.4Hz, 2H, pyr-$CH_2$),

4.4-3.8 (m, 7H),

3.14 (q, J=6Hz, 2H, $CH_2N$),

1.7-0.7 (m, 29H aprox.).

*Analysis* calcd. for $C_{28}H_{47}N_3O_6$: C 64.46%; H 9.08%; N 8.05%.

Found: C 64.43%; K 8.80%; N 7.87%.

EXAMPLE 13

2(*N*-Acetyl-*N*-(((2-pentadecylcarbamoyloxymethyl-1,3-dioxolan-4-yl)methoxy) carbonyl) aminomethyl) pyridine.

Following the procedure described in example 2, but using the product prepared in example 12, instead of the one prepared in example 1, the title compound was obtained as a dense colorless liquid in a similar yield.

IR (film) ν:

3319, 2916, 2845, 1730, 1686, 1591, 1536, 1463, 1428, 1367, 1270, 1221, 1151;

$^1$H NMR (80 MHz, $CDCl_3$) δ (TMS):

8.50 (broad d, J=4Hz, 1H, pyr.),

7.64 (dt, $J_d$=1.8HZ, $J_t$=7.5Hz, 1H, pyr),

7.3-7.0 (m, 2H, pyr),

5.08 (s, 2H, pyr-$CH_2$),

5.05 (t, J=4.5Hz, 1H, OCHO),

4.7 (broad s, 1H, NH),

4.3-3.4 (m, 7H),

3.14 (q, J=6Hz, 2H, $CH_2N$),

2.62 (s, 3H, O=$CCH_3$),

1.7-0.7 (m, 29H aprox.).

EXAMPLE 14

2(*N*-Acetyl-*N*-(((2-pentadecylcarbamoyloxymethyl-1,3-dioxolan-4-yl)methoxy) carbonyl) aminomethyl)-1-ethylpyridinium iodide.

Following the procedure described in example 3, but using the product prepared in example 13 and ethyl iodide, instead of the one prepared in example 2 and methyl iodide, the title compound was obtained as a yellow solid in a similar yield.
mp: 38-40 °C;
IR (KBr) $\nu_{max}$ ($cm^{-1}$):
3399, 2920, 2848, 1755, 1701, 1625, 1579, 1513, 1448, 1367, 1222, 1162, 1138, 1088, 1042;
$^1H$ NMR (80 MHz, $CDCl_3$) $\delta$ (TMS):
9.54 (d, J=6Hz, 1H, pyr),
8.53 (t, J=7.5Hz, 1H, pyr),
8.05 (t, J=6.5Hz, 1H, pyr),
7.85 (d, J=8Hz, 1H, pyr),
5.50 (s, 2H, pyr-$CH_2$),
5.3-4.9 (m, 4H, OCHO, NH, NEt),
4.6-4.2 (m, 3H),
4.2-3.8 (m, 4H),
3.13 (q, J=6Hz, 2H, $CH_2N$),
2.67 (s, 3H, O=$CCH_3$),
1.73 (t, J=7Hz, 3H, NEt),
1.7-0.7 (m, 29H aprox.)
*Analysis* calcd. for $C_{32}H_{54}IN_3O_7$ : C 53.40%; H 7.56%; N 5.84%.
Found: C 53.18%; H 7.88%; N 5.66%.

In the following Formulations are described the preparation of a number of pharmaceutical formulations in accordance with the invention.

## FORMULATION 1

### Capsules

A well blended mixture of 100 mg of powdery active compound, 197 mg of lactose, and 3 mg of magnesium stearate is encapsulated in a hard gelatin capsule which is washed and dried.

## FORMULATION 2

### Tablets

A mixture of 100 mg of the active compound, 2.5 mg of magnesium stearate, 125 mg of dibasic calcium phosphate, 10 mg of sodium starch glycolate and 12.5 mg of talc is compounded and tableted.

## FORMULATION 3

### Injectable solutions

100 mg of the active compound is mixed with 0.05 ml of benzylic alcohol and 1 ml of propylene glycol. The mixture is made up with sterilized water and then sterilized.

FORMULATION 4

Creams

5g parts of a base cream is prepared from 40% white petrolatum, 10% lanolin, 5% Span 20, 0.3% Tween 20 and 41.7% water, with which 100 mg of a powdery active compound are admixed.

FORMULATION 5

Coating liquids

1 part by weight of the active compound is mixed with 99 parts by weight of polyethylene glycol 300.

FORMULATION 6

Ointments

2 parts of the active compound, 40 parts of polyethylene glycol 400 and 58 parts by weight of polyethylene glycol 1500 are mixed and solubilized, with heating, and then cooled.

FORMULATION 7

Aerosol

4 g of the active compound is mixed with 100 ml of propylenglycol and 0.2 g of an aromatizer. The mixture is made up with a suitable propellant.

FORMULATION 8

Syrup

0.4 g of the active substance is mixed with 45 g of sacharose, 0.2 g of an aromatizer and 0.1 g of sodium saccharine. The mixture is made up with water to 100 ml.

The compounds of the present invention have valuable PAF antagonist activity, which may be used both for the treatment and protection of animals, including human beings, as illustrated by the following Experiments:

EXPERIMENT 1

Inhibition of platelet aggregation induced by PAF.

The blood is obtained by cardiac puncture of male New Zealand albino rabbits (between 2 and 2.5 Kg of weight) and coagulation is prevented by adding 1 part of 3.16% sodium citrate dihydrate in 9 parts of blood. The platelet rich plasma (PRP) is prepared by blood centrifugation at 250xg for 10 min. at 4° C and it is diluted with platelet poor plasma (PPP) by additional centrifugation at 3000xg for 10 min. The amount of platelets is adjusted to $3x10^{-5}/mm^3$. The platelet aggregation induced by PAF ($C_{18}$; prepared in our laboratory) (16 nM, final) is determined by the Born nephelometric technique (*J. Physiol.*, **1962**, 162, 67) using a aggregometer Chrono-log 500. The activities of the inhibitors are expressed as $IC_{50}$, that is to say the concentration of the drug needed to inhibit the platelet aggregation by 50%. The results are shown in table 1:

Table 1.

| Compound number | $IC_{50}$ (μM) |
|---|---|
| 3 | 2.2 |
| 4 | 0.21 |
| 7 | <0.1 |
| 10 | 1.2 |
| 11 | 0.16 |
| 14 | <0.1 |

EXPERIMENT 2

Inhibition of the hypotensive effect induced by PAF in normotense rats.

Male Sprage Dawley rats, of 180-220 g of weight, anesthetized with sodium pentobarbital (50 mg/Kg, i.p. 1 mL/100 g) have been used. In order to measure the average arterial pressure, a polyethylene catheter is introduced into the carotid artery. The arterial pressure is recorded with the help of a transducer connected with a R611 Beckman polygraph. The tested compounds are administered through the femoral vein 3 min before the injection of PAF (0.5 mcg/Kg, i.v.). The inhibition of the hypotension induced by PAF of the different compounds expressed as $IC_{50}$, is shown in table 2.

Table 2.

| Compound number | $CI_{50}$ (mg/Kg,i.v.) |
|---|---|
| 3 | 0.9 |
| 4 | 0.2 |
| 7 | <0.1 |
| 10 | 0.35 |
| 11 | <0.1 |
| 14 | <0.1 |

## Claims

1). Compounds of Formula (**I**)

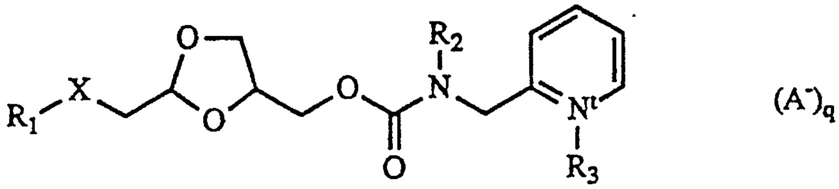

(I)

wherein,

$R_1$ represents a $C_{10}$-$C_{24}$ alkyl, alkenyl or alkynyl group, a *tert*-butyl group, an aryl-$C_1$-$C_{12}$ alkyl or a cyclohexyl-$C_1$-$C_{12}$ alkyl group;

$R_2$ is H or a -C(=O)$R_4$ group, wherein $R_4$ represents $C_1$-$C_6$ alkyl or $C_1$-$C_6$ alkoxy;

X is a single bond or a -O-, -C(=O)O- or -$NR_5$C(=O)O- group, wherein $R_5$ is H, $C_1$-$C_4$-alkyl or $C_1$-$C_4$ acyl;

q is 0 or 1;

t is (+) when q=1, and t has no meaning when q=0;

$R_3$ is H or $C_1$-$C_6$ alkyl when q=1 and an electron pair when q=0;

$A^-$ is a pharmaceutically acceptable anion.

2). Compounds as claimed in Claim 1, wherein:

$R_1$ represents a $C_{10}$-$C_{24}$ alkyl group, a *tert*-butyl group, an aryl-$C_1$-$C_6$ alkyl group or a cyclohexyl-$C_1$-$C_6$-alkyl group;

$R_2$ is hydrogen or a -C(=O)$R_4$ group, wherein $R_4$ represents $C_1$-$C_3$ alkyl or $C_1$-$C_3$ alkoxy;

X is a single bond or -O-, or -$NR_5$C(=O)O-, wherein $R_5$ is hydrogen, $C_1$-$C_4$ alkyl or $C_1$-$C_4$ acyl;

q is 0 or 1;

t is (+) when q=1, and t has no meaning when q=0;

$R_3$ is hydrogen or $C_1$-$C_3$ alkyl when q=1 and it is an electron pair when q=0;

$A^-$ is a pharmaceutically acceptable ion.

3). Compounds as claimed in Claim 2, wherein:

$R_1$ represents a $C_{10}$-$C_{20}$ alkyl group;

$R_2$ is a -C(=O)$R_4$ group, wherein $R_4$ represents $C_1$-$C_3$ alkyl or $C_1$-$C_3$ alkoxy;

X is a single bond or -O-, or -NHC(=O)O-;

q is 1;

t is (+);

$R_3$ is ethyl or propyl;

$A^-$ is iodide or chloride.

4). Compounds as claimed in Claim 3, wherein:

$R_2$ is a -C(=O)$CH_3$ group;

$R_3$ is ethyl;

$R_1$, X, q, t, and $A^-$ are as defined in claim 3.

5).2-(*N*-acetyl-*N*-(((2-heptadecyl-1,3-dioxolan-4-yl) methoxy) carbonyl)aminomethyl)-1-ethylpyridinium iodide.

6).2(*N*-acetyl-*N*-(((2-hexadecyloxymethyl-1,3-dioxolan-4-yl) methoxy)carbonyl) aminomethyl)-1-ethylpyridinium iodide

7). 2-(*N*-acetyl-*N*-(((2-pentadecylcarbamoyloxymethyl-1,3-dioxolan-4-yl) methoxy) carbonyl) aminomethyl)-1-ethylpyridinium iodide.

8). A pharmaceutical composition comprising a pharmaceutically acceptable carrier or diluent in admixture with at least one compound of formula (I)

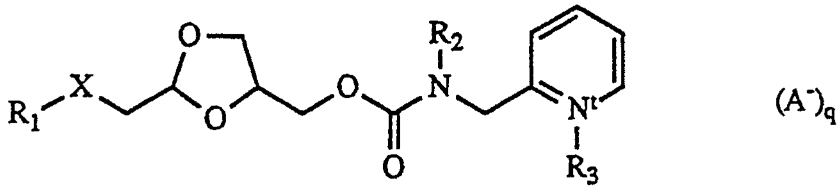

(I)

wherein,
$R_1$ represents a $C_{10}$-$C_{24}$ alkyl, alkenyl or alkynyl group, a *tert*-butyl group, an aryl-$C_1$-$C_{12}$ alkyl or a cyclohexyl-$C_1$-$C_{12}$ alkyl group;
$R_2$ is H or a -C(=O)$R_4$ group, wherein $R_4$ represents $C_1$-$C_6$ alkyl or $C_1$-$C_6$ alkoxy;
X is a single bond or a -O-, -C(=O)O- or -$NR_5$C(=O)O- group, wherein $R_5$ is H, $C_1$-$C_4$ alkyl, or $C_1$-$C_4$ acyl;
q is 0 or 1
t is (+) when q=1 and t has no meaning when q = 0;
$R_3$ is H or $C_1$-$C_6$ alkyl when q=1 and an electron pair when q=0;
$A^-$ is a pharmaceutically acceptable anion.

9). A composition according to Claim 8 in which said compound is a compound according to anyone of Claims 1 to 7.

10). A composition according to Claim 8 in which said compound is a compound according to anyone of Claims 5 to 7.

11). A process for preparing a compound according to any one of Claims 1 to 7, which process comprises:
(a) reacting 1-benzyloxy-2,3-propanediol (**II**), with a compound of formula (**III**)
$R_1$-X-$CH_2$CHO (**III**)
[in which $R_1$ and X are as defined above] to give a compound of formula (**IV**)

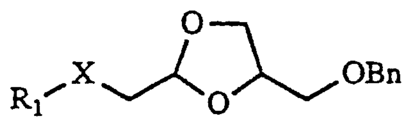

(IV)

[in which $R_1$ and X are as defined above] and reacting said compound of formula (**IV**) with hydrogen gas in the presence of a metal catalyst to give compound (**V**)

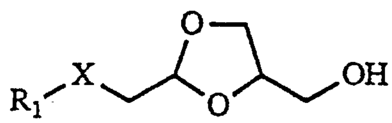

(V)

[in which $R_1$ and X are as defined above] and reacting said compound of formula (**V**) with a compound of formula (**VI**)
$Y'C(=O)Y''$ (**VI**)
[in which $Y'$ and $Y''$ are good leaving groups] to give a compound of formula (**VII**)

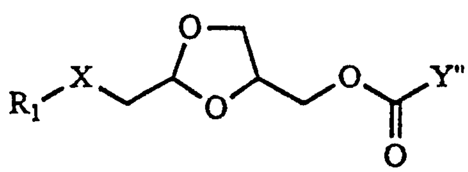

(VII)

[in which $R_1$, $Y''$ and X are as defined above] and reacting said compound of formula (**VII**) with 2-aminomethylpyridine to afford a compound of formula (**Ia**)

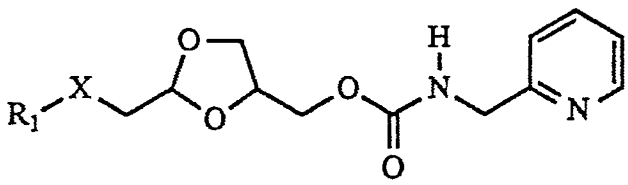

(Ia)

[in which $R_1$ and X are as defined above] and optionally reacting said compound of formula (**Ia**) with a compound of formula (**VIII**)

$ClC(=O)R_4$ (**VIII**)

[in which $R_4$ is as defined above] to give a compound of formula (**Ib**)

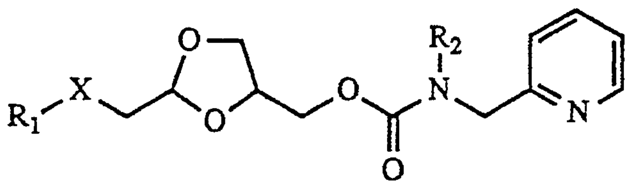

(Ib)

[in which $R_1$, $R_2$, and X are as defined above, but $R_2$ is different from hydrogen] and optionally reacting said compound (**Ib**)with a compound of formula (**IX**)

$R_3A$ (**IX**)

[in which $R_3$ and A are as defined above] to afford a compound of formula (**Id**)

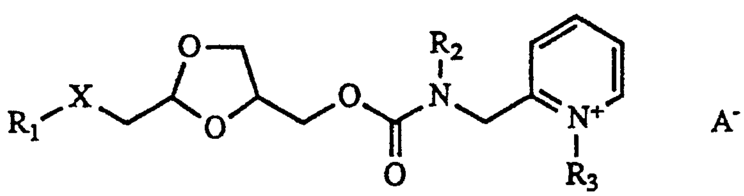

(Id)

[in which $R_1$, $R_2$, $R_3$, X, and A are as defined above, but $R_2$ is different from hydrogen];

or

(b) optionally reacting a compound of formula (**Ia**) with a compound of formula (**IX**) to give a compound of formula (**Ic**)

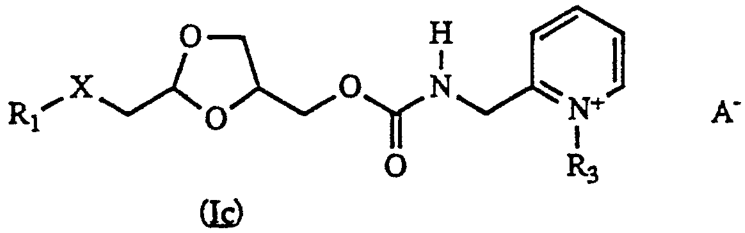

(Ic)

[in which $R_1$, $R_3$, X, and A are as defined above].

and

(c) optionally changing anion A by another pharmaceutically acceptable anion by means of ion-exchange chromatography or selective salt precipitation.

12. The use for the manufacture of a medicament for the treatment or prophylaxys of PAF-mediated illnesses of a compound of formula (**I**), as defined in Claim 9.

13. The use according to Claim 12 of a compound according to any one of Claims 1 to 7.

Claims for the following Contracting State: GR

1. A process for preparing a compound of formula (I):

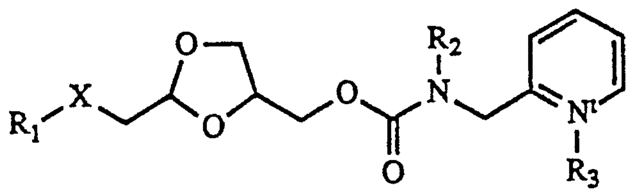

$(A^-)_q$

(I)

wherein,
$R_1$ represents a $C_{10}$-$C_{24}$ alkyl, alkenyl or alkynyl group, a *tert*-butyl group, an aryl-$C_1$-$C_{12}$ alkyl or a cyclohexyl-$C_1$-$C_{12}$ alkyl group;
$R_2$ is H or a -C(=O)$R_4$ group, wherein $R_4$ represents $C_1$-$C_6$ alkyl or $C_1$-$C_6$ alkoxide;
X is a single bond or a -O-, -C(=O)O- or -$NR_5$C(=O)O- group, wherein $R_5$ is H, $C_1$-$C_4$ alkyl, or $C_1$-$C_4$ acyl;
q is 0 or 1
t is (+) when q=1 and t has no meaning when q =0; $R_3$ is H or $C_1$-$C_6$ alkyl when q=1 and an electron pair when q=0;
$A^-$ is a pharmaceutically acceptable anion,
which process comprises:
(a) reacting 1-benzyloxy-2,3-propanediol (II), with a compound of formula (III)
$R_1$-X-$CH_2$CHO (III)
[in which $R_1$ and X are as defined above] to give a compound of formula (IV)

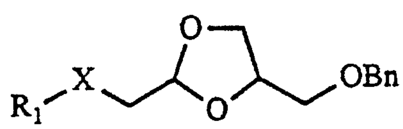

(IV)

[in which $R_1$ and X are as defined above] and reacting said compound of formula (IV) with hydrogen gas in the presence of a metal catalyst to give compound (V)

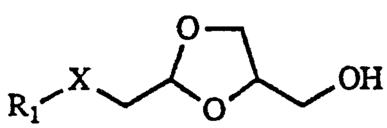

(V)

[in which $R_1$ and X are as defined above] and reacting said compound of formula (V) with a compound of formula (VI)
$Y'C(=O)Y''$ (VI)
[in which $Y'$ and $Y''$ are good leaving groups] to give a compound of formula (VII)

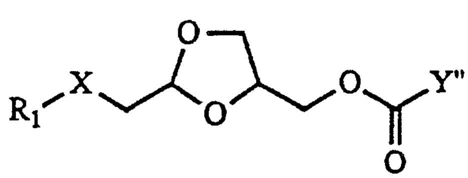

(VII)

[in which $R_1$, $Y''$ and X are as defined above] and reacting said compound of formula (**VII**) with 2-aminomethylpyridine to afford a compound of formula (**Ia**)

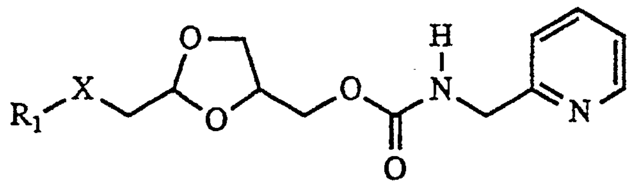

(Ia)

[in which $R_1$ and X are as defined above] and optionally reacting said compound of formula (**Ia**) with a compound of formula (**VIII**)

$ClC(=O)R_4$ (**VIII**)

[in which $R_4$ is as defined above] to give a compound of formula (**Ib**)

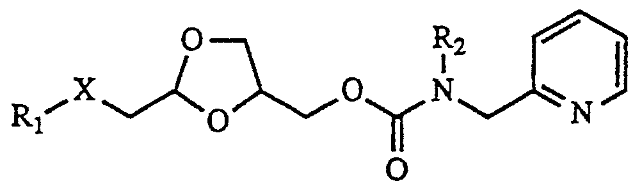

(Ib)

[in which $R_1$, $R_2$, and X are as defined above, but $R_2$ is different from hydrogen] and optionally reacting said compound (**Ib**) with a compound of formula (**IX**)

$R_3A$ (**IX**)

[in which $R_3$ and A are as defined above] to afford a compound of formula (**Id**)

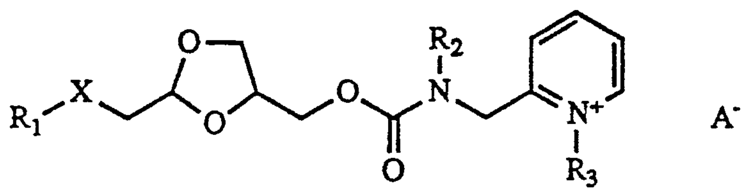

(Id)

[in which $R_1$, $R_2$, $R_3$, X, and A are as defined above, but $R_2$ is different from hydrogen];

or

(b) optionally reacting a compound of formula (**Ia**) with a compound of formula (**IX**) to give a compound of formula (**Ic**)

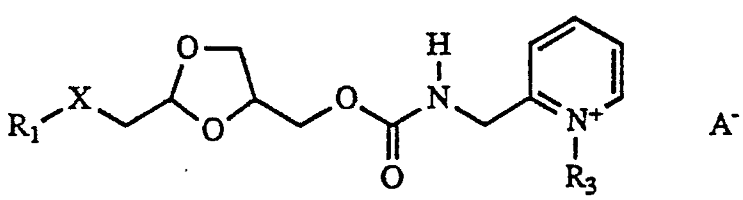

(Ic)

[in which $R_1$, $R_3$, X, and A are as defined above].

and

(c) optionally changing anion A by another pharmaceutically acceptable anion by means of ion-exchange

chromatography or selective salt precipitation.

2. A process according to Claim 1 which the reagents and reaction conditions are so chosen as to prepare a compound of formula (**I**) in which:

$R_1$ represents a $C_{10}$-$C_{24}$ alkyl group, a *tert*-butyl group, an aryl-$C_1$-$C_6$ alkyl group or a cyclohexyl-$C_1$-$C_6$-alkyl group;

$R_2$ is hydrogen or a -C(=O)$R_4$ group, wherein $R_4$ represents $C_1$-$C_3$ alkyl or $C_1$-$C_3$ alkoxide;

X is a single bond or -O-, or -$NR_5$C(=O)O-, wherein $R_5$ is hydrogen, $C_1$-$C_4$ alkyl or $C_1$-$C_4$ acyl;

q is 0 or 1;

t is (+) when q=1, and t has no meaning when q=0;

$R_3$ is hydrogen or $C_1$-$C_3$ alkyl when q=1 and it is an electron pair when q=0;

$A^-$ is a pharmaceutically acceptable ion.

3. A process according to Claim 2 in which the reagents and reaction conditions are so chosen as to prepare a compound of formula (**I**) in which:

$R_1$ represents a $C_{10}$-$C_{20}$ alkyl group;

$R_2$ is a -C(=O)$R_4$ group, wherein $R_4$ represents $C_1$-$C_3$ alkyl or $C_1$-$C_3$ alkoxide;

X is a single bond or -O-, or -NHC(=O)O-;

q is 1;

t is (+);

$R_3$ is ethyl or propyl;

$A^-$ is iodide or chloride.

4. A process according to Claim 2 in which the reagents and reaction conditions are so chosen as to prepare a compound of formula (**I**) in which:

$R_2$ is a -C(=O)$CH_3$ group;

$R_3$ is ethyl;

$R_1$, X, q, t, and $A^-$ are as defined in claim 3.

5. A process according to Claim 1 in which the reagents and reaction conditions are so chosen as to prepare 2-(*N*-acetyl-*N*-(((2-heptadecyl-1,3-dioxolan-4-yl) methoxy) carbonyl) aminomethyl)-1-ethylpyridinium iodide.

6. A process according to Claim 1 in which the reagents and reaction conditions are so chosen as to prepare 2-(*N*-acetyl-*N*-(((2-hexadecyloxymethyl-1,3-dioxolan-yl) methoxy) carbonyl) aminomethyl)-1-ethylpyridinium iodide.

7. A process according to Claim 1 in which the reagents and reaction conditions are so chosen as to prepare 2-(*N*-acetyl-*N*-(((2-pentadecylcarbamoyloxymethyl-1,3-dioxolan-4-yl) methoxy) carbonyl)-aminomethyl)-1-ethylpyridinium iodide.